# EUROPEAN PATENT APPLICATION

(11) **EP 4 797 669 A1**
(43) Date of publication of application: **26.08.2026**
(21) Application number: 24942084.5
(22) Date of filing: 30.12.2024
(51) Int. Cl.: H04M 1/72454

(54) **SURGICAL INSTRUMENT**

(71) Applicant: Hangzhou Mindray Medical Technology Co., Ltd., Hangzhou, Zhejiang 311508 (CN)
(72) Inventor: ZHANG, Yangyang, Beijing 100176 (CN); BI, Jiashuai, Beijing 100176 (CN); ZHANG, Honglei, Beijing 100176 (CN); LU, Yiming, Beijing 100176 (CN); QIAO, Yong, Beijing 100176 (CN); LI, Yingjie, Beijing 100176 (CN)
(74) Representative: KIPA AB
(86) International application number: PCT/CN2024/143976
(87) International publication number: WO 2026/143383

(57) **Abstract**

A surgical instrument is disclosed, including: an end effector assembly, which includes a first jaw and a second jaw closed to clamp a tissue of a patient; a staple cartridge assembly between the first jaw and the second jaw, which includes multiple stitching staples and a firing member to fire the staples and/or cut the tissue; a drive system, which includes a drive source and a transmission mechanism; the drive source provides a drive force to drive the firing member to move via the transmission mechanism, so as to fire the staples and/or cut the tissue; and a controller for determining a firing speed of the firing member based on a time parameter of a closing process of the jaws. The firing speed is a movement speed, when firing the staple(s) and/or cutting the tissue. The surgical instrument protects tissue effectively and improves yield of stitching staple.

## Description

### TECHNICAL FIELD

The disclosure relates to a technical field of medical instrument, and more particularly to a surgical instrument.

### BACKGROUND

At present, a minimally invasive surgery occupies an important position in surgeries, and a surgical stapling instrument is a commonly used surgical instrument in a minimally invasive surgery. A working principle of the surgical stapling instrument is to clamp a jaw of an end effector at a designated position adjacent a lesion site, excise a tissue through a cutting knife of the end effector, and stitch through a staple cartridge assembly of the end effector while excising.

The surgical stapling instrument uses a firing member to fire stitching staples, so as to eject the stitching staples for stitching a tissue. A Chinese patent application with a public number CN117257380A uses a time parameter, which is related to tissue squeezing, as a basis for speed control, when firing the stitching staples, a good cutting/stitching effect on a tissue can be achieved. But in some cases, introducing more parameters as a basis for firing is beneficial for better handling various situations, further improving a yield rate of stitching staples, and protecting a tissue.

### SUMMARY

According to a first aspect, some embodiments provide a surgical instrument, including:
an end effector assembly, which includes a first jaw and a second jaw, which are configured to clamp a tissue of a patient, when the first jaw and the second jaw are closed;
a staple cartridge assembly, which is arranged between the first jaw and the second jaw, wherein the staple cartridge assembly includes multiple stitching staples, and a firing member which is configured to fire the multiple stitching staples and/or cut the tissue of the patient;
a drive system, which includes a drive source and a transmission mechanism; wherein the drive source is configured to provide a drive force to drive the firing member to move via the transmission mechanism, so as to fire the multiple stitching staples and/or cut the tissue of the patient; and
a controller, which is at least configured to determine a firing speed of the firing member, based on a time parameter of a closing process of the first jaw and the second jaw, wherein the firing speed is a movement speed of the firing member, when the firing member fires the stitching staple(s) and/or cuts the tissue of the patient.

According to another aspect, some embodiments provide a surgical instrument, including:
an end effector assembly, which includes a first jaw and a second jaw, which are configured to clamp and squeeze a tissue of a patient;
a staple cartridge assembly, which is arranged between the first jaw and the second jaw, wherein the staple cartridge assembly includes multiple stitching staples, and a firing member which is configured to fire the multiple stitching staples and/or cut the tissue of the patient;
a drive system, which includes a drive source and a transmission mechanism; wherein the drive source is configured to provide a drive force to drive the firing member to move via the transmission mechanism, so as to fire the multiple stitching staples and/or cut the tissue of the patient; and
a controller, which is at least configured to determine a firing speed of the firing member, when the firing member fires the stitching staple(s) and/or cuts the tissue of the patient, based on a closing speed and/or a clamping force of the first jaw and the second jaw during a closing process of the first jaw and the second jaw.

According to a surgical instrument of an above embodiment, a movement speed of a firing member, when the firing member fires a stitching staple and/or cuts, is determined, based on at least one of a time parameter, a closing speed and a clamping force during a closing process of a first jaw and a second jaw. By using the time parameter, the closing speed and the clamping force during a closing process of the first jaw and the second jaw, a thickness and a type of a tissue, as well as a firing strategy expected by a doctor, can be determined, thereby achieving a better yield of stitching staple and a better stitching effect.

### BRIEF DESCRIPTION OF THE DRAWINGS

FIG. 1 is a structural diagram of a surgical instrument according to an embodiment.
FIG. 2 is a structural diagram of an end effector assembly according to an embodiment.
FIG. 3 is a structural diagram a staple cartridge assembly according to an embodiment.
FIG. 4 is a structural diagram a staple cartridge assembly according to an embodiment.

100. Transmission mechanism;
200. End effector assembly; 210. First jaw; 220. Second jaw;
300. Staple cartridge assembly; 310. Mouting seat;
312. Tissue contacting surface; 313. Movable cavity; 314. Support portion; 315. Position-limiting portion;
320, Firing member; 330. Cutting knife;
400. Handle assembly; 410. Fixed handle; 420. Movable trigger;
500. Barrel assembly.

### DESCRIPTION OF THE PREFERRED EMBODIMENTS

This disclosure is further described in detail below through specific embodiments in combination with the accompanying drawings. In different embodiments, similar elements adopt associated similar reference numbers. In the following embodiments, many details are described so as to make this disclosure better understood. However, one skilled in the art can easily recognize that some of the features can be omitted in different cases, or can be replaced by other elements, materials and methods. In some cases, some operations related to this disclosure are not shown or described in the description, so as to avoid a core part of this disclosure being inundated by too many descriptions. For one skilled in the art, it is not necessary to describe these related operations in detail, as they can fully understand the relevant operations according to the description in this disclosure and the general technical knowledge in the art.

In addition, the features, operations, or characteristics described in this disclosure may be combined in any appropriate manner to form various embodiments. At the same time, the steps or actions in the method description can also be exchanged or regulated in order in a manner obvious to one skilled in the art. Therefore, the various sequences in the description and the drawings are only for the purpose of clearly describing a certain embodiment, and do not mean that they are necessary sequences, unless it is otherwise specified that one of the sequences must be followed.

The terms "first", "second", etc. in the description are used to distinguish different objects, rather than to describe a specific order. In addition, the terms "connection", and "linkage" mentioned in this disclosure include direct and indirect connection (linkage), unless otherwise specified.

The inventor found that by using a time parameter related to tissue squeezing as a basis for determining a movement speed of a firing member, a yield rate of stitching staples can be effectively improved when dealing with many tissues, especially thick tissues. However, when facing some special tissues, relying solely on this parameter as a basis has certain shortcomings. Specifically, when facing tissues, such as pancreas, hardened liver, or lung lobes, doctor needs to take a special care of these tissues due to their fragility or hardness, so as to prevent damage caused by a closing process. When a special care is needed for a tissue, in order to reduce a damage caused by closing to the tissue, jaws are slowly closed at a lower speed while the jaws are closed. Usually, after closing to a certain extent, a time for squeezing the tissue will be longer, thus slowly squeezing the tissue and allowing the tissue to flow slowly without damaging the tissue. At the same time, the firing speed should be controlled to prevent tissue damage. When facing these tissues, there are certain shortcomings in considering time after starting clamping, that is, taking a squeezing time as a whole, or only considering time after clamping a tissue to a characteristic position, has certain shortcomings. If for the above tissues, only adjust the firing speed by a squeezing time, it is likely to be determined as a thin tissue and needs to use a faster speed for firing. However, the actual situation is not like this. For tissues that require a special care, doctor needs to use a slower firing speed to fire. Therefore, embodiments of this disclosure propose to determine a type of tissue based on a closing process, and further adjust the firing speed based on these parameters combining the squeezing time, in order to comprehensively consider various different scenarios and determine a more accurate firing speed.

"Distal end" in this disclosure refers to an end which is away from an operator when said operator operates the surgical instrument, and a "proximal end" refers to an end which is adjacent to an operator when said operator operates the surgical instrument. "Axial direction" in this disclosure refers to a direction of a connection line between a centre of the distal end and a centre of the proximal end, and "radial direction" refers to a direction which is perpendicular to the axial direction.

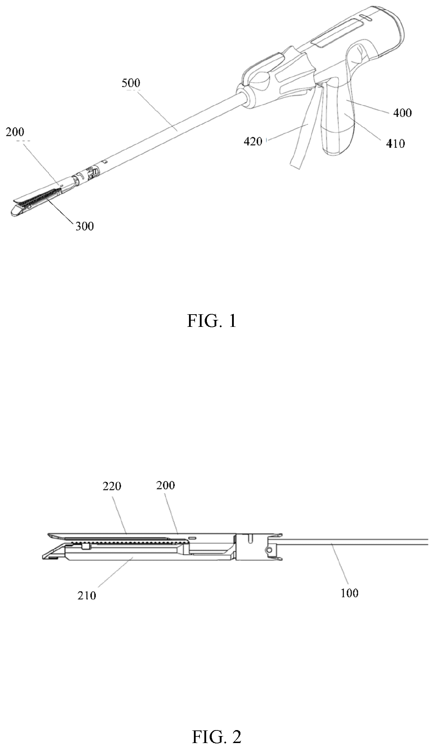

Please refer to an embodiment shown in FIGS. 1 to 4, which provide a surgical instrument, including a transmission mechanism 100, an end effector assembly 200, a staple cartridge assembly 300, a drive source and a controller (the drive source and controller are not shown). For example, the surgical instrument can be a surgical stapling instrument.

In some embodiments, the surgical instrument may further include a handle assembly 400 and a barrel assembly 500. The handle assembly 400 includes a fixed handle 410 and at least one movable trigger 420 for an operator to hold and operate. A proximal end of barrel assembly 100 is connected with the handle assembly 400, and a distal end of barrel assembly 500 is connected with the end effector assembly 200. The barrel assembly 500 includes a closed transmission chain and a firing transmission chain. When operating the surgical instrument, an operator can hold the fixed handle 410 and insert the end effector assembly 200 into a lesion site inside a human body through the barrel assembly 500 which is slender, and then trigger the closed transmission chain through the movable trigger 420 to close jaws of the end effector assembly 200 to clamp adjacent to a lesion site. Then, a firing button (not shown) is configured to trigger the firing transmission chain, so as to enable a cutting knife 330 inside the end effector assembly 200 to cut a tissue, and at the same time, to stitch the tissue by stitching staples of the staple cartridge assembly 300.

In some embodiments, the surgical instrument may not include the handle assembly 400, and the proximal end of the barrel assembly 500 can be connected with another assembly, such as a robotic arm or a manipulator of the surgical robot, so as to insert the end effector assembly 200 into the lesion site inside the human body through the barrel assembly 500, and then close the transmission chain and trigger the firing transmission chain through a mechanical/electrical manner, so as to complete jaw closing, tissue cutting and stitching operations.

In some embodiments, the firing transmission chain includes a transmission mechanism 100, which is configured to, under an action of a drive source, implement firing of the stitching staples and/or cutting of the cutting knife 330.

The end effector assembly 200 includes a first jaw 210 and a second jaw 220, which are configured to clamp a tissue. The first jaw 210 and the second jaw 220 are in movable connection with each other, wherein the first jaw 210 and the second jaw 220 are capable of moving relative to each other, so as to close and open. A mounting cavity is formed between the first jaw 210 and the second jaw 220, and the staple cartridge assembly 300 is mounted inside the mounting cavity. A distal end of the transmission mechanism 100 extends into the mounting cavity. A squeezing involves applying a certain force to a tissue by the first jaw 210 and the second jaw 220. As a certain force is applied to the tissue during a closing process. In this application, a stage of compression after closing is mainly referred to as squeezing.

In some embodiments, a tissue gradually deforms over time under a squeezing force of jaws until the tissue reaches equilibrium, i.e., a creep characteristics of the tissue reaches equilibrium. When an external force disappears, the tissue tends to recover to its original state. Accordingly, the longer a squeezing time, the smaller an internal stress inside the tissue, and the greater a deformation of the tissue.

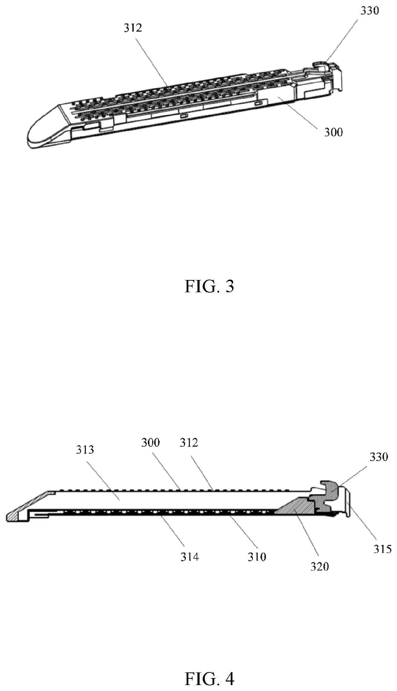

Please continue to refer to FIGS. 3 and 4. The staple cartridge assembly 300 includes a mounting seat 310, a firing member 320, and a cutting knife 330. The mounting seat 310 is provided with multiple mounting positions for mounting stitching staples, and the mounting seat 310 is further provided with a tissue contacting surface 312 for contacting a tissue. The firing member 320 is movably arranged at the mounting seat 310, and the cutting knife 330 is movably connected with the mounting seat 310. The firing member 320 and the cutting knife 330 are capable of moving between a proximal end and a distal end of the mounting seat 310. The transmission mechanism 100 is capable of driving the firing member 320 and the cutting knife 330 to move between the proximal end and the distal end of the mounting seat 310. It should be noted that, the tissue contacting surface 312 of the mounting seat 310 refers to a surface of the mounting seat 310 that comes into contact with the tissue being clamped, while clamping the tissue. It can be understood that during stitching a tissue, a stitching staple is pushed out from the tissue contacting surface 312 of the mounting seat 310 to stitch the tissue. Pushing out a stitching staple refers to firing a stitching staple by the firing member 320. In some embodiments, the cutting knife 330 is a portion of the firing member 320, so as to enable the firing member 320 to cut a tissue of a patient through the cutting knife 330.

In some embodiments, the mounting base 310 is provided with a movable cavity 313, which is arranged along an axial direction of the mounting base 310, wherein the firing member 320 and the cutting knife 330 are movable within the movable cavity 313. Respective movement directions of the firing member 320 and the cutting knife 330 are limited by the movable cavity 313 to ensure that the firing member 320 and the cutting knife 330 can move along the axial direction of the mounting seat 310 between the proximal end and the distal end of the mounting seat 310.

In some embodiments, the mounting base 310 is provided with a support portion 314 located on a side of the movable chamber 313, which side back-faces the mounting position, for supporting the firing member 320 and the cutting knife 330. The support portion 314 prevents the firing member 320 and the cutting knife 330 from being separated from the movable chamber 313 on a side of the mounting seat 310, which side back-faces the mounting position.

In some embodiments, a proximal end of the mounting base 310 is provided with a position-limiting portion 315, which is configured to prevent the firing member 320 and the cutting knife 330 from being separated from the movable cavity 313 from the proximal end of the mounting base 310. The position-limiting portion 315 prevents the firing member 320 and the cutting knife 330 from being separated from the movable cavity 313 from the proximal end of the mounting seat 310.

In some embodiments, the staple cartridge assembly 300 can be considered as a portion of the end effector assembly 200. For example, the staple cartridge assembly 300 can be fixedly connected with the first jaw 210 or the second jaw 220, or may form an integral structure with the first jaw 210 or the second jaw 220. The staple cartridge assembly 300 can also be considered as a separate assembly independent of the end effector assembly 200. For example, the staple cartridge assembly 300 can be detachably mounted at the first jaw 210 or the second jaw 220, so as to enable the staple cartridge assembly 300 to be regard as a consumable applied to the end effector assembly 200.

The drive source is capable of driving, via the transmission mechanism 100, the firing member 320 to move. Specifically, the drive source can apply a force along an axial direction of the transmission mechanism 100 to move the transmission mechanism 100 towards a distal end, so as to enable the transmission mechanism 100 to push the firing member 320 to move. The firing member 320 fires one or more stitching staples to stitch a tissue, while the firing member 320 moves. It can be understood that, when the drive source drives the transmission mechanism 100 with different drive parameters, speeds of the transmission mechanism 100 are different, so that movement speeds of the firing member 320 and firing speeds of the stitching staple are also respectively different. In some embodiments, the drive source is a motor, and the speed of the transmission mechanism 100 can be controlled by setting a parameter, such as a current and a rotation speed and other parameters, of the motor.

In this embodiment, a start position and an end position can be defined in a stroke of the firing member 320 for moving from a proximal end to a distal end, and a process between the start position and the end position is defined as a firing process. A movement speed from the start position to the end position represents a firing speed. In some embodiments, the start position refers to a position where the firing member 320 starts to fire the stitching staple(s), while the firing member 320 moves; the end position refers to a position where the firing member 320 locates, when a tissue stitching has been completed. In some embodiments, a position, where the firing member 320 fires part or all of the stitching staples in the mounting base 410, is defined as the end position. In other embodiments, the end position is defined by a distance, and a preset distance exists between the end position and the start position. It can be understood that the faster the firing member 320 moves from the start position to the end position, the faster the firing process ends, the faster the firing speed of the entire process, and the faster the stitching speed of the tissue. Conversely, the slower the firing member 320 moves from the start position to the end position, the slower the firing process ends, the slower the firing speed of the entire process, and the slower the stitching speed of the tissue. The key concept of this embodiment lies in how to control a movement speed of the firing member 320 from the start position to the end position. It can be understood that this is also equivalent to how to control a firing speed of the stitching staple, or how to control a drive parameter of a drive source, or how to control a stitching speed of tissue.

The controller is at least configured to determine a firing speed of the firing member, based on a time parameter of a closing process of the first jaw 210 and the second jaw 220. The firing speed of the firing member is a movement speed of the firing member when the firing member fires the stitching staple(s) and/or cuts the tissue of the patient. It can be understood that the controller controls the movement speed of the transmission mechanism 100 and thus controls the movement speed of the firing member 320 by controlling a drive parameter of the drive source. In some embodiments, the controller may also determine the movement speed of the firing member 320 when the firing member 320 fires the stitching staple(s), based on a time parameter when the first jaw 210 and the second jaw 220 are closed, that is, to determine a firing speed for the firing member to fire the stitching staple(s) based on respective time parameters of a closing process and of a squeezing process of tissue after the closing process. In an embodiment of this disclosure, the controller controls the movement speed of the firing member 320 in two aspects according to time parameter(s). The movement speed in the first aspect is a firing speed when the firing member 320 starts to fire the stitching staple, that is, an initial movement speed of the firing member 320 at a start position. The movement speed in the second aspect is a movement speed of the firing member 320 while the firing member 320 fires the stitching staple(s). For example, the controller can continuously adjust the movement speed of the firing member 320 from the start position to the end position. The following two aspects of implementation examples will be explained separately.

In some embodiments, the surgical instrument further includes a timing device, which is configured to determine a time parameter during a closing process of the first jaw 210 and the second jaw 220, as well as a time parameter during a tissue squeezing process of the first jaw 210 and the second jaw 220 after the closing process. The timing device may be a portion of the controller or an external component of the controller. In some embodiments, the time parameter of the closing process includes a closing time, which is determined by the controller based on when the first jaw 210 and the second jaw 220 are at respective first relative positions and when the first jaw 210 and the second jaw 220 are at respective second relative positions, such as a closing time for the first jaw 210 and the second jaw 220 to be closed from their respective first relative positions to their respective second relative positions. The timing device can start timing when the first jaw 210 and the second jaw 220 are at their respective first relative positions, and record a closing time when the first jaw 210 and the second jaw 220 are at their respective second relative positions. The timing device can pause after recording the closing time. The respective first relative positions and the respective second relative positions can be customized by a user according to a situation, or can be some relative positions that satisfy a preset condition. In some embodiments, the time parameter of the closing process includes a time from when the first jaw 210 and the second jaw 220 start to close until when the first jaw 210 and the second jaw 220 are closed and locked. A closed and locked state is a state, in which the first jaw 210 and the second jaw 220 are locked in their respective relative positions, and the transmission mechanism is restricted from moving in a direction for opening the first jaw 310 and the second jaw 320, so as to maintain the first jaw and the second jaw to be closed. In an example, the movable trigger 420 can be a closing trigger used for closing the jaws. When the closing trigger is operated to limit, the jaws will automatically lock after completing closing, so as to protect the jaws in a closed state. In some embodiments, in the closed and locked state, a user can unlock and reopen the jaws by pressing a button.

The Chinese patent application with an application number CN202310577378.7 describes a surgical stapling instrument with structure(s) for detecting closing and for releasing closing and locking, which can be adopted this disclosure.

The respective first relative positions include but are not limited to following positions.
1. The respective first relative positions can be respective positions, at which the first jaw 210 and the second jaw 220 start to clamp a tissue. For example, both the first jaw 210 and the second jaw 220 are respectively provided with one pressure sensor. When a certain value appears on both pressure sensors, it means that both the first jaw 210 and the second jaw 220 are in contact with the tissue, and then the timing apparatus obtains a time parameter through timing. It is understood that whether the first jaw 210 and the second jaw 220 start to clamp the tissue can also be determined by other means.
2. The respective first relative positions can be respective positions, at which the first jaw 210 and the second jaw 220 are closed to a preset distance from each other, or the first jaw 210 and the second jaw 220 are closed to a preset angle. For example, a distance sensor is arranged between the first jaw 210 and the second jaw 220. When the first jaw 210 and the second jaw 220 are closed to a preset distance from each other, the timing apparatus obtains a time parameter through timing after then. For another example, a movable connection position of the first jaw 210 and the second jaw 220 is provided with an angle measurement apparatus. When the first jaw 210 and the second jaw 220 are closed to a preset angle, the timing apparatus obtains a time parameter through timing after then.
3. The respective first relative positions can be respective positions, at which the first jaw 210 and the second jaw 220 simultaneously satisfy starting to clamp a tissue and being closed to a preset distance/angle. For example, the distance sensor/the preset angle sensor, detects that the first jaw 210 and the second jaw 220 are closed to a preset distance/angle, and both pressure sensors show a certain value, at said position.

The respective second relative positions include but are not limited to following positions.
1. The respective second relative positions can be relative positions at which the first jaw 210 and the second jaw 220 clamp a tissue to a designated position. For example, the first jaw 210 and the second jaw 220 are respectively provided with a pressure sensor. When a measurement result of the pressure sensor exceeds a preset pressure threshold, it means that the first jaw 210 and the second jaw 220 clamp the tissue to a designated position, and then the timing apparatus obtains a time parameter through timing. It is understood that whether the first jaw 210 and the second jaw 220 clamp the tissue to a designated position can also be determined by other ways.
2. The respective second relative positions can be respective positions, at which the first jaw 210 and the second jaw 220 are closed to a preset distance from each other, or the first jaw 210 and the second jaw 220 are closed to a preset angle.
3. The respective second relative positions can be respective positions, at which the first jaw 210 and the second jaw 220 simultaneously satisfy clamping a tissue to a designated position and being closed to a preset distance/angle.

It should be noted that, timing starts when the first jaw 210 and the second jaw 220 are at their respective first relative positions, and recording a closing time when the first jaw 210 and the second jaw 220 are at their respective second relative positions, refer to timing, based on when the first jaw and the second jaw are at the respective first relative positions or when the first jaw and the second jaw are the respective second relative positions, rather than necessarily timing immediately when the first jaw and the second jaw are at the respective first relative positions or the respective second relative positions. For example, the timing can also start to obtain the time parameter after the first jaw 210 and the second jaw 220 has been closed to a designated relative position for a certain time length T. It is also possible to start timing after performing a closing-related operation at their respective first relative positions or their respective second relative positions, such as start timing after performing a closing and locking operation at their respective second relative positions.

To clarify meaning and effect of the respective first relative positions and the respective second relative positions, several examples will be used to illustrate. For example, when the respective first relative positions are positions, at which the first jaw 210 and the second jaw 220 starts to clamp a tissue, and the respective second relative positions are positions, at which the first jaw 210 and the second jaw 220 clamp the tissue to a designated position, timing starts from when starting to clamp the tissue until when clamping the tissue to the designated position (such as when measurement results of pressure sensors show a certain value or exceed a preset pressure threshold), so as to obtain a closing time. When treating with a tissue that require a special care, a closing speed for such tissue after contacting said tissue is usually slower to prevent damage to said tissue while closing, which results in a longer closing time. However, when facing a tissue that does not require a special care, a closing speed for such tissue is quick and then the closed tissue can be squeezed to prevent damage to said tissues. Therefore, the controller can adjust a firing speed based on a length of the closing time. By detecting a relative position at a start of closing and an end of closing, the closing time can be accurately determined.

For example, when the respective first relative positions are positions, at which the first jaw 210 and the second jaw 220 are closed to a first preset angle, and the respective second relative positions are positions, at which the first jaw 210 and the second jaw 220 are closed to a second preset angle, timing starts from the first preset angle to the second preset angle, so as to record the closing time. The first preset angle can be set according to a situation, for example, the first preset angle can be an average angle at which jaws contact the tissue. In some cases, to further prevent damage to the tissue caused by closing, or to ensure that the jaws are clamped at a correct position of the tissue, a doctor may close the jaws to a certain degree and reopen the jaws for multiple times, and the jaws may be separated from the tissue while being opened. Start timing after closing to a preset starting angle can prevent a problem of re-timing due to separation of the jaws from the tissue. When the jaws are closed to the second preset angle, it can be considered that the closing has been completed at said angle, or a time for closing to said angle is sufficient as a basis for determination. The example of the first jaw 210 and the second jaw 220 to be closed to a preset distance is similar to the example of to the preset angle.

For example, the respective first relative positions are positions, at which the first jaw 210 and the second jaw 220 simultaneously satisfy starting to clamp a tissue and be closed to a preset angle, the respective second relative positions are positions, at which the first jaw 210 and the second jaw 220 simultaneously satisfy clamping a tissue to a designated position and being closed to a preset distance/angle. At this time, an angle sensor detects that the first jaw 210 and the second jaw 220 are closed to the preset angle, and both pressure sensors show corresponding values. For example, the respective first relative positions are positions, at which the first jaw 210 and the second jaw 220 starts to clamp a tissue, and the respective second positions are positions, at which the first jaw 210 and the second jaw 220 are closed to a preset angle. In the above examples, many detailed descriptions have been made to make the closing time of the respective first relative positions and the respective second relative positions easier to understand, rather than requiring compliance with these descriptions, nor does it mean that only the above examples exist. In some embodiments, a third relative position or more relative positions are also included to obtain more parameters to assist in controlling the firing member to fire the stitching staple, such as setting a closing start position, a semi-closed position, a complete-closed position, etc.

A type of a closed tissue can be determined by the controller based on a closing time, which is determined by the respective first relative positions and the respective second relative positions of the first jaw 210 and the second jaw 220. In some embodiments, in order to prevent tissue damage during the closing process, a slow closing is usually performed to prolong the closing time and reduce the firing speed of the firing member 320, so as to protect the tissue. Therefore, the longer the closing time, the more the tissue needs to be protected, and the lower the firing speed of the firing member 320. Therefore, in this embodiment, the controller controls the firing speed of the firing member 320 to be in a negative correlation with the closing time. This negative correlation is limited within a certain range, such as within a preset speed range.

In some embodiments, preset time limits can be set as a comparison basis for determining a type of a tissue. For example, when the closing time is less than a first preset time, a currently operated tissue is treated as a regular tissue. When the closing time is greater than the first preset time, a currently operated tissue is treated as a tissue that needs protection during closing. For example, when the closing time is less than the first preset time, a degree of a negative correlation between the closing time and the firing speed of the firing member 320 is reduced. When the closing time is greater than the first preset time, at least one of the following can be reasonably set: a range of an initial speed, when the firing member 320 starts to fire the stitching staple and/or cut a tissue of a patient; a degree of a negative correlation between the closing time and the initial speed; a range of a movement speed, while the firing member 320 fires a stitching staple and/or cuts a tissue of a patient; and a degree of a negative correlation between the movement speed, while the firing member 320 fires the stitching staple and/or cuts a tissue of a patient, and the closing time; so as to prevent tissue damage caused by too fast firing. The range of the initial speed or the range of the movement speed while the firing member 320 fires the stitching staple can include a maximum value and a minimum value. Setting the maximum value can prevent tissue damage, while setting the minimum value can reduce unnecessary waiting time. When the maximum value and the minimum value are equal, it is equivalent to setting the speed to a constant value. The degree of a negative correlation refers to a rate of change for speeds, when the closing time changes. In an example, a relationship curve exists between the closing time and the firing speed of the firing member 320, and a slope of each point on the relationship curve can indicate a rate of change for speeds. For example, more preset times can be set to make the firing member 320 more suitable for various situations to fire the stitching staple. For example, at different preset times, ranges of movement speeds of the firing member 320, while the firing member 320 fires the stitching staple and/or cuts the tissue of the patient, are different; or under a certain preset time, a movement speed of the firing member 320, while the firing member 320 fires the stitching staple and/or cuts the tissue of the patient, is a constant value.

In some embodiments, the first preset time is set, and whether the closing time is greater than the first preset time is used a basis to determine a type of a tissue, and then the firing speed of the firing member 320 is controlled in combination with a squeezing-related time. That is, the controller is configured to determine whether a current tissue needs a special care, based on whether a closing time is greater than a first preset time, wherein the closing time is determined based on when the first jaw 210 and the second jaw 220 are at their respective first relative positions and when the first jaw 210 and the second jaw 220 are at their respective second relative positions; and to comprehensively control a firing speed of the firing member 320 based on a squeezing-related time parameter after the first jaw 210 and the second jaw 220 are at their respective second relative positions, such as a pre-squeezing time from when the first jaw 210 and the second jaw 220 are closed to their respective second relative positions until when the firing member 320 starts to fire stitching staple(s). When the closing time is greater than the first preset time, the controller controls an initial speed, when the firing member 320 starts to fire the stitching staple(s) and/or cut the tissue of the patient, to be in a negative correlation with a sum of the closing time and the pre-squeezing time, so as to make the initial speed smaller to protect the tissue. At the same time, the controller controls a movement speed, while the firing member 320 fires the stitching staple and/or cuts the tissue of the patient, such as a range of speed or a specific value of speed. When the closing time is less than the first preset time, the closing time is in a negative correlation with the initial speed, and the pre-squeezing time is in a positive correlation with the initial speed, so as not to spend too much firing time on a tissue that does not require protection. The squeezing-related time parameter after the first jaw 210 and the second jaw 220 are at their respective second relative positions may include a squeezed time in which the tissue continues to be clamped, when the firing member 320 fires the stitching staple. The movement speed of the firing member 320, while the firing member 320 fires the stitching staple and/or cuts the tissue of the patient, can be determined by the closing time, and the movement speed can be adjusted in combination with the squeezed time. For example, a controller can be configured to control the movement speed of the firing member 320, while the firing member 320 fires the stitching staple and/or cuts the tissue of the patient, to be in a negative correlation with a sum of the closing time and the squeezed time. For example, when the squeezed time is sufficient to protect the tissue, the movement speed of the firing member 320, while the firing member 320 fires the stitching staple, can be maintained at a constant value. It should be noted that the squeezed time is a dynamically changing parameter, which can also be seen as a sum of a pre-squeezing time and a fired time.

In addition to the closing time, a closing speed of the first jaw 210 and the second jaw 220 during a closing process of the first jaw 210 and the second jaw 220 can also be obtained. For example, a distance sensor is arranged between the first jaw 210 and the second jaw 220, and the controller calculates the closing speed based on data collected by the two distance sensors within the closing time. For example, an angle measurement device is arranged at a moveable connection point between the first jaw 210 and the second jaw 220, and the controller obtains the closing speed based on the data collected by the angle measurement device within the closing time. When facing a tissue that requires a special care, the closing speed is usually relatively slow. The change of the closing speed can also be used to determine the type of tissue and control the firing speed of the firing member 320. In some embodiments, the obtained closing speed is continuous, while in other embodiments, the closing speed can also be obtained at intervals during the closing process, i.e., the obtained closing speed is discrete.

In some embodiments, the controller controls the firing speed of the firing member 320 based on a rate of change of the closing speed within the closing time. For example, a relationship curve of the closing speed over time is generated based on continuous or discrete closing speeds, and a slope of the relationship curve can represent the rate of change of the closing speed. After obtaining the relationship curve of change, the firing speed of the firing member 320 is determined based on the relationship curve of change. In some embodiments, statistical value(s) of the closing speed within the closing time can also be obtained to determine the firing speed of the firing member 320 based on the statistical value(s). The statistical value(s) of the closing speed include(s) but is(are) not limited to an average value, a maximum value, etc.

In some embodiments, a clamping force of the first jaw 210 and the second jaw 220 during the closing process can also be obtained. For example, both the first jaw 210 and the second jaw 220 are respectively provided with one pressure sensor, and the controller obtains pressure values collected by the two pressure sensors within the closing time as the clamping force(s). Of course, it is also possible to only mount a pressure sensor at the first jaw 210 or the second jaw 220. When facing a tissue that require a special care, it is usually necessary to close with a small force to prevent damage to the tissue caused by a large clamping force. Therefore, the clamping force during the closing process can be used to determine a type of the tissue and control the firing speed of the firing member 320. In some embodiments, the obtained clamping force is continuous, while in other embodiments, the clamping force can also be obtained at regular intervals during the closing process, i.e., the obtained clamping force is discrete.

In some embodiments, the controller determines the firing speed of the firing member 320 based on a rate of change of the clamping force within the closing time. For example, a relationship curve of clamping force over time is generated based on continuous or discrete clamping forces, and the slope of the relationship curve can indicate the rate of change of the clamping force. After obtaining the relationship curve of change, the firing speed of the firing member 320 is determined based on the relationship curve of change. In some embodiments, statistical value(s) of the clamping force within the closing time can also be obtained to determine the firing speed of the firing member 320 based on the statistical value(s). The statistical value(s) of the clamping force include(s) but is(are) not limited to an average value, a maximum value, etc.

Although the principles herein have been shown in various embodiments, many modifications of structures, arrangements, proportions, elements, materials, and assemblies particularly suitable for the specific environmental and operational requirements may be used without departing from the principles and scope of the present disclosure. The above modifications and other changes or amendments are included in the scope of this disclosure.

The foregoing specific description has been described with reference to various embodiments. However, those skilled in the art recognize that various modifications and changes can be made without departing from the scope of the present disclosure. Therefore, consideration of the present disclosure is in an illustrative rather than a restrictive sense, and all such modifications are included within the scope thereof. Also, the advantages of various embodiments, other advantages, and the solutions to problems have been described above. However, the benefits, advantages, solutions to problems, and any elements that can produce these, or solutions that make them more explicit, should not be interpreted as critical, necessary, or essential. The term "including", and any other variants thereof used herein are non-exclusive, so that the process, method, document, or device that includes a list of elements includes not only these elements, but also other elements that are not explicitly listed or do not belong to the process, method, system, document, or device. Furthermore, the term "coupling" and any other variations thereof used herein refer to physical connection, electrical connection, magnetic connection, optical connection, communicational connection, functional connection, and/or any other connection.

## Claims

1. A surgical instrument, **characterized in that**, comprising:
an end effector assembly, which comprises a first jaw and a second jaw, which are configured to clamp a tissue of a patient, when the first jaw and the second jaw are closed;
a staple cartridge assembly, which is arranged between the first jaw and the second jaw, wherein the staple cartridge assembly comprises multiple stitching staples, and a firing member which is configured to fire the multiple stitching staples and/or cut the tissue of the patient;
a drive system, which comprises a drive source and a transmission mechanism; wherein the drive source is configured to provide a drive force to drive the firing member to move via the transmission mechanism, so as to fire the multiple stitching staples and/or cut the tissue of the patient; and
a controller, which is at least configured to determine a firing speed of the firing member, based on a time parameter of a closing process of the first jaw and the second jaw, wherein the firing speed is a movement speed of the firing member, when the firing member fires the stitching staple(s) and/or cuts the tissue of the patient.

2. The surgical instrument according to claim 1, **characterized in that**, the firing speed comprises:
an initial speed, when the firing member starts to fire the stitching staple(s) and/or cut the tissue of the patient; and/or
a movement speed, while the firing member fires the stitching staple(s) and/or cuts the tissue of the patient.

3. The surgical instrument according to claim 2, **characterized in that**, the time parameter of the closing process comprises a closing time, which is determined by the controller based on when the first jaw and the second jaw are at respective first relative positions and when the first jaw and the second jaw are at respective second relative positions;
wherein the controller is further configured to determine the firing speed at least based on the closing time.

4. The surgical instrument according to claim 3, **characterized in that**, the closing time, which is determined by the controller based on when the first jaw and the second jaw are at respective first relative positions and when the first jaw and the second jaw are at respective second relative positions, comprises:
a closing time for the first jaw and the second jaw to be closed from their respective first relative positions to their respective second relative positions.

5. The surgical instrument according to claim 3, **characterized in that**, the controller is further configured to control the firing speed of the firing member to be in a negative correlation with the closing time.

6. The surgical instrument according to claim 3, **characterized in that**, the first jaw and the second jaw are further configured to squeeze the tissue of the patient;
wherein the controller is further configured to control the initial speed, when the firing member starts to fire the stitching staple(s) and/or cut the tissue of the patient, to be in a negative correlation with a sum of the closing time and a pre-squeezing time, when the closing time is greater than a first preset time;
wherein the pre-squeezing time is a time from when the first jaw and the second jaw are closed to their respective second relative positions until when the firing member starts to fire the stitching staple(s).

7. The surgical instrument according to claim 3, **characterized in that**, in order to determine the firing speed at least based on the closing time, the controller is specifically configured to:
obtain a closing speed of the first jaw and the second jaw during the closing process; and
determine the firing speed based on the closing time and the closing speed during the closing process.

8. The surgical instrument according to claim 7, **characterized in that**, the controller configured to determining the firing speed based on the closing time and the closing speed during the closing process, comprises: the controller configured to:
obtain a rate of change of the closing speed within the closing time, and determine the firing speed based on said rate of change; or
obtain a statistical value of the closing speed during the closing process, and determine the firing speed based on said statistical value.

9. The surgical instrument according to claim 3, **characterized in that**, in order to determine the firing speed at least based on the closing time, the controller is specifically configured to:
obtain a clamping force of the first jaw and the second jaw during the closing process; and
determine the firing speed based on the closing time and the clamping force during the closing process.

10. The surgical instrument according to claim 9, **characterized in that**, the controller configured to determining the firing speed based on the closing time and the clamping force during the closing process, comprises: the controller configured to:
obtain a rate of change of the clamping force within the closing time, and determine the firing speed based on said rate of change; or
obtain a statistical value of the clamping force during the closing process, and determine the firing speed based on said statistical value.

11. The surgical instrument according to claim 2, **characterized in that**, the time parameter of the closing process comprises a closing time from when the first jaw and the second jaw start to close until when the first jaw and the second jaw are closed and locked.

12. A surgical instrument, **characterized in that**, comprising:
an end effector assembly, which comprises a first jaw and a second jaw, which are configured to clamp and squeeze a tissue of a patient;
a staple cartridge assembly, which is arranged between the first jaw and the second jaw, wherein the staple cartridge assembly comprises multiple stitching staples, and a firing member which is configured to fire the multiple stitching staples and/or cut the tissue of the patient;
a drive system, which comprises a drive source and a transmission mechanism; wherein the drive source is configured to provide a drive force to drive the firing member to move via the transmission mechanism, so as to fire the multiple stitching staples and/or cut the tissue of the patient; and
a controller, which is at least configured to determine a firing speed of the firing member, when the firing member fires the stitching staple(s) and/or cuts the tissue of the patient, based on a closing speed and/or a clamping force of the first jaw and the second jaw during a closing process of the first jaw and the second jaw.
